# EUROPEAN PATENT SPECIFICATION

(11) **EP 0 768 867 B1**
(45) Date of publication and mention of the grant of the patent: **09.10.2002**
(21) Application number: 94920203.0
(22) Date of filing: 13.06.1994
(51) Int. Cl.: A61K 9/00

(54) **DOSAGE FORM FOR ADMINISTERING DRUG IN LIQUID FORMULATION**
DOSIERUNGSFORM ZUR VERABREICHUNG VON FLÜSSIGE ARZNEIMITTEL FORMULIERUNG
FORME GALENIQUE POUR L'ADMINISTRATION D'UN MEDICAMENT DANS UNE FORMULATION LIQUIDE

(43) Date of publication of application: 23.04.1997
(73) Proprietor: ALZA CORPORATION, Palo Alto California 94303-0802 (US)
(72) Inventor: LARSEN, Steven, D., Dublin, CA 94568 (US); GUITTARD, George, V., Cupertino, CA 95014 (US); WONG, Patrick, S.-L., Palo Alto, CA 94303 (US)
(74) Representative: Stebbing, Peter John Hunter
(86) International application number: US9406723
(87) International publication number: WO95034285

(56) References cited:
- WO-A-91/15196
- GB-A- 2 155 889

## Description

### FIELD OF THE INVENTION

This invention relates to a dosage form comprising a non-aqueous liquid formulation comprising a drug for oral administration from the dosage form to an aqueous biological environment of use. In particular, the invention pertains to a dosage form comprising a non-aqueous liquid formulation comprising a drug that does not lend itself to administration by conventional liquid formulations. More preferably, the invention concerns a dosage form comprising means for administering an aqueous-sensitive drug at a controlled-release rate in an effective dose for improved bioavailability by administering the drug in a non-aqueous liquid formulation for protecting the drug from potential degradation and for enhancing drug absorption.

### BACKGROUND OF THE INVENTION

Aqueous-liquid formulations conventionally are used in medicine for administering drug to an aqueous-biological environment of use. Conventional aqueous formulations are provided as ready-made formulations and as reconstitutable formulations for administering of a drug. With both of these formulations, there are many drugs that cannot be administered by these formulations because they require a non-aqueous liquid formulation. Moreover, non-aqueous liquid formulations are not common in the dispensing art. Then too, the dispensing art does not provide a dosage form for the controlled-release of a non-aqueous liquid formulation comprising a liquid-sensitive drug in an effective dose at a rate-controlled over time. For example, cyclosporine, an immunosuppressive useful in organ transplantation, currently is administered by injection, because the drug is practically insoluble in water and consequently, it does not lend itself for formulation into a conventional aqueous formulation for oral administration. Another drug in need of an improved oral-aqueous liquid dosage form is captopril, an angiotensin converting enzyme inhibitor. A non-aqueous liquid dosage form is needed for this drug because it is adversely affected by oxidation, and if a non-aqueous liquid formulation is provided for protecting the drug for preventing oxidation, the dosage form would be an improvement in the dispensing art. Presently, the dispensing art lack a dosage form that can simultaneously house and protect a liquid drug formulation prior to its delivery from the dosage form.

Another shortcoming associated with the prior art dosage form, manufactured as a capsule containing a drug, is the drug is present in a non-release rate, non-aqueous liquid carrier prior to and after its release from the capsule. That is, capsules containing an non-aqueous drug formulation dispense, when the capsule breaks open, all the drug immediately into the environment with the consequence that, the drug concentration is high followed by a total absence of drug between later administered capsules. For many drugs, this form of peak drug administration followed by a low valley or absence of drug, can have undesirable effects, especially if the drug possesses a low therapeutic index, if the drug is not suited to time-varying rates of administration, and if the therapy requires continuous and controlled drug delivery.

It will be appreciated by those versed in the dispensing art in the light of the above presentation, that if a dosage form can be provided that is free of the tribulations known to the prior art, such dosage forms would have a positive, practical value, and it would represent also an advancement in the dispensing art. Likewise, it will be immediately appreciated by those versed in the dispensing art, that if a dosage form is made available that possesses thermodynamic activity for dispensing a non-aqueous liquid drug formulation at a controlled rate, such a dosage form would find a practical application in the fields of medicine and pharmacy.

WO 91/15196 provides a dosage form comprising an outer semipermeable wall (12), a middle osmotically active layer (13) and a capsule (14) spaced from the wall (12). GB-A-2155889 provides an osmotic dispenser containing a heat responsive composition.

Accordingly, it is an immediate object of this invention to provide a dosage form comprising means for dispensing a liquid aqueous-free drug formulation at a controlled rate over a prolonged period of time, with the dosage form representing both a improvement and an advancement in the liquid formulation dispensing art.

Another object of the invention is to provide a non-aqueous based liquid formulation for dispensing from a prolonged administration dosage form, which combination of dosage form an non-aqueous liquid formulation possesses good shelf-life and improved bioavailability of the dispensed drug.

Another object of the invention is to provide a non-aqueous liquid formulation for oral administration comprising an active drug which formulation can be rate-dispensed over a prolonged time at a dose-effective rate for this drug's therapy.

Another object of the invention is to provide a dosage form designed and adapted for dispensing a drug in a non-aqueous lipophilic formulation as a controlled and known rate over a prolonged time, to a patient in need of the drug-liquid formulation.

Another object of the invention is to provide a dosage from comprising a drug that is difficult to delivery because of its properties, but can be delivered in a lipophilic liquid-formulation by a controlled means, which means is at least one of a number selected from sustained-release and prolonged-release dosage forms.

Anotherobject of the invention is to provide a dosage from possessing at least one of a member selected from the group consisting of, a sustained-release, and substantially zero-order prolonged-release dosage form, which dosage form comprises means for high loading of a water insoluble drug or a practically water insoluble drug, and which dosage form comprises means for delivering the drug in a lipophilic-liquid formulation over time to a drug recipient.

Another object of the invention is to provide a dosage form comprising means for delivering a liquid-drug formulation that comprises a drug and a non-toxic, biologically acceptable carrier, than surrounds the drug, and which carrier provides protection from oxidation and provides an independent pH uninfluenced by the environment of use for the continuous dispensing of the drug over an extended period of time up to thirty hours.

Another object of the invention is to provide a dosage form comprising a lipophilic liquid carrier for a drug, which carrier exhibits dispensable properties at room temperature and dispensable properties at mammalian temperatures for facilitating drug delivery in a therapeutically effective dose over a prolonged time.

Another object of the invention is to provide for the administration of a drug from an oral dosage form that possesses the kinetic ability to dispense a lipophilic drug formulation over a broad range of drug delivery rates, and which method can deliver the drug at a sustained-release or controlled-prolonged-release rate over time.

Another object of the invention is to provide a dosage form sized, shaped and adapted for oral, buccal, sublingual, vaginal or anorectal drug delivery, which dosage form comprises a drug-liquid formulation comprising one of a member selected from the group consisting of a monoglyceride, a diglyceride, a triglyceride, and a glycerol ester of a fatty acid for compounding a lipophilic liquid drug formulation that can be dispensed from an osmotic dosage form over time.

Another object of the invention is to provide a dosage form comprising a non-aqueous lipophilic drug formulation which lipophilic drug formulation is essentially-free of reaction with the dosage form and can be dispensed at a bioreceptive rate.

Other objects, features, aspects and advantages of the invention will be more apparent to those skilled in a the drug dispensing art from the following detailed specification, taken in conjunction with the drawings and the accompanying claims.

According to the present invention therefore there is provided a dosage form (10) for the administration of a non-aqueous lipophilic liquid drug composition (22) to an environment of use, wherein the dosage form comprises:
(a) an outside wall (12) comprising a polymer semipermeable to the passage of a biological fluid and substantially impermeable to the passage of the drug;
(b) an inside wall (19) in contact with outside wall (12), said inside wall (19) comprising a protein polymer;
(c) a compartment (20) formed by the outside and the inside wall together;
(d) a hydrophilic push composition (26) in the compartment;
(e) an exit (13) in the outside and inside walls that connects the exterior with the inside of the dosage form;
(f) a non-aqueous lipophilic liquid drug (22) composition in the compartment;
(g) said liquid drug composition comprising a drug and a carrier (21) for the drug comprising a member selected from a hydrogenated glyceride, a monoglyceride, a diglyceride, and a triglyceride; a member selected from a glycol, a polyalkylene glycol and an ester of a glycol; and a hydrogenated oil;
characterised in that there is provided a substantially fluid impermeable barrier layer (27) positioned between the push composition (26) and the drug composition (22) and in that the drug is a non-aqueous liquid at both room temperature and at mammalian temperature to facilitate delivery of a therapeutically effective dose therefrom over time.

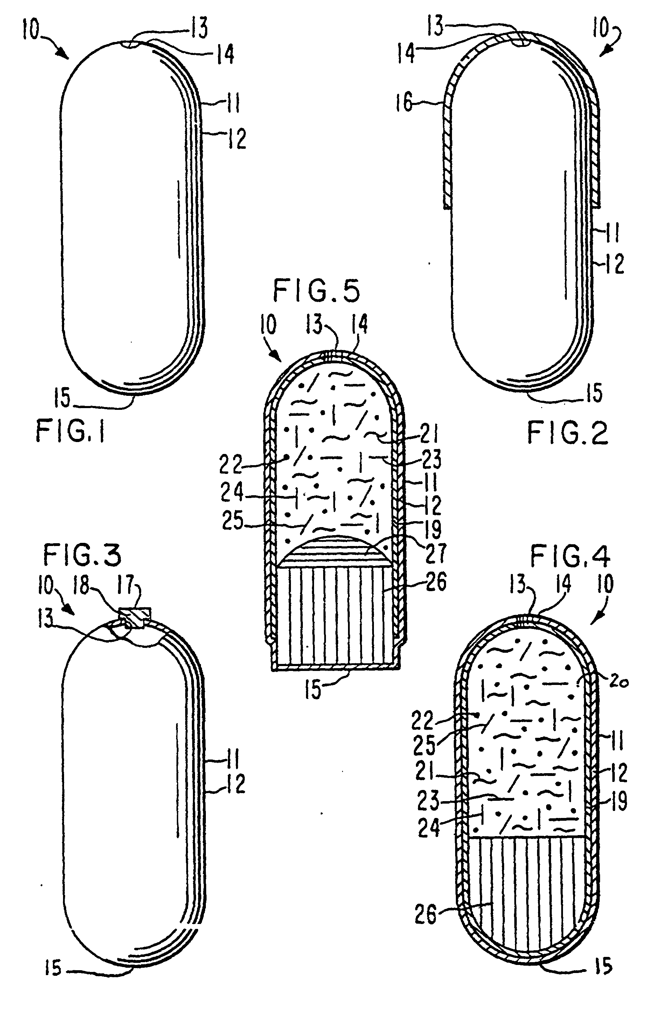

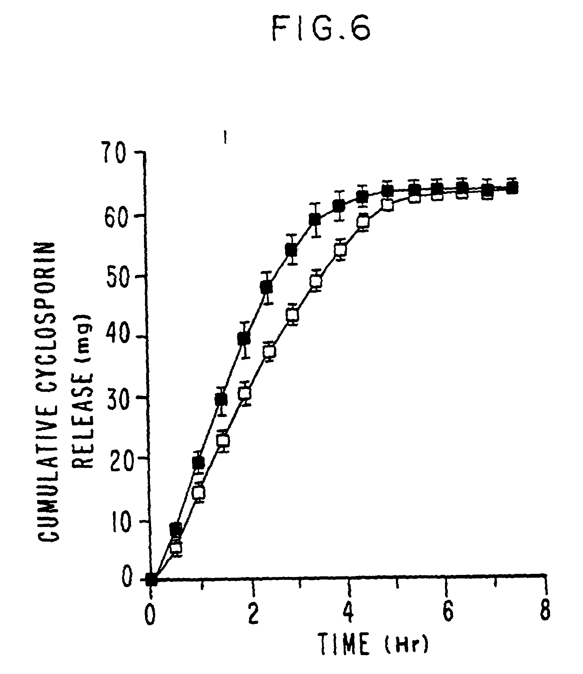

In the drawing figures, which are not drawn to scale, but are set forth to illustrate various embodiments of the invention, the drawing figures are as follows:
Drawing Figure 1 is a general view of a dosage form provided by this invention;
Drawing Figure 2 is a view of drawing Figure 1, with the addition of a cap that closes the exit port of the dosage form;
Drawing Figure 3 is a view of drawing Figure 1, with means for sealing the exit port in the exit port;
Drawing Figure 4 is an opened view of drawing Figure 1, which depicts the physical structure comprising a non-aqueous liquid drug formulation and a driving means for pushing the liquid drug formulation from the dosage form;
Drawing Figure 5 is an opened view of drawing Figure 1, which drawing Figure 5 depicts the dosage form comprising an internal two-walled structure; and,
Drawing Figure 6 is a graph depicting the release rate pattern for two dosage forms provided by the invention.

In the drawings and in the specification, like parts in related figures are identified by like parts. The terms appearing earlier in the specification, and in the description of the drawing figures, as well as embodiments thereof, are further detailed elsewhere in the disclosure.

### DETAILED DISCLOSURE OF DRAWING FIGURES

Turning now to the drawing figures in detail, which drawing figures are examples of dosage forms provided by the invention, and which examples are not to be construed as limiting, one example of a dosage form is seen in drawing Figure 1. In drawing Figure 1, dosage form 10, is seen comprising a body 11, which body 11 comprising a wall 12 that surrounds an internal lumen, not seen in drawing Figure 1. Dosage form 10 comprises an exit passageway 13, and a lead end 14 distant from rear end 15. Exit passageway 13 connects the exterior of dosage form 10 with the interior of dosage form 10.

Drawing Figure 2 illustrates dosage form 10 comprising body 11, wall 12, exit means 13, lead end 14 and rear end 15. Drawing Figure 2 comprises additionally closure means 16 that covers exit means 13 for preventing a premature delivery or leakage of fluid from inside dosage form 10. Closure means 16 covers exit means 13 with closure means 16 in contact arrangement over part of lead end 14. Closure means 16 comprises a material impermeable to the passage of fluid such as high density fluid impermeable polyolefin including high density polyethylene. The closure means 16 possesses a shape that corresponds to the external shape of wall 12, and it can be applied by dipping, laminating, coating, spraying or by similar manufacture techniques.

Drawing Figure 3 illustrates dosage form 10 comprising body 11, wall 12, exit means 13, lead end 14 and rear end 15. Drawing Figure 3 additionally comprises in dosage form 10 an exit closure 17 position in exit port 13. Exit closure 17 is a stopper that halts or causes to stop fluid flow from dosage form 10. The closure 17 shuts exit 13 and it includes a bung, a cork, an impermeable plug made by an impermeable material such as aluminized polyethylene, rubber, silicon, nylon, synthetic fluorine teflon, chlorinated hydrocarbon polyolefins, and fluorinated vinyl polymers. Closure 17 extends into exit port 13, or closure 17 extends into exit port 13 with its shoulders 18 extended over lead end 14.

In drawing Figure 4, wall
12 surrounds an inner wall 19 that forms an inner capsule 19 with an inner lumen or space 20. Wall 12 is substantially impermeable to the passage of drug 22. Wall 12 comprises a semipermeable composition that does not adversely affect an animal or a host, and the selectively semipermeable compositions used for forming semipermeable wall 12 are non-erodible and they are insoluble aqueous fluids. Representative compositions for forming the semipermeable wall 12 include semipermeable homopolymers, semipermeable copolymers, semipermeable block copolymers, and the like. In one embodiment, typical semipermeable polymers comprise cellulose esters, cellulose ethers, and cellulose ester-ethers. These cellulosic polymers have a degree of substitution, D.S., on their anyhydroglucose unit from greater than 0 up to 3 inclusive. By degree of substitution is meant the average number of hydroxyl groups originally present on the anyhydroglucose unit that are replaced by a substituting group, or converted into another group. The anhydroglucose unit can be partially or completely substituted with groups such as acyl, alkanoyl, alkenoyl, aroyl, alkyl, alkoxy, halogen, carboalkyl, alkylcarbamate, alkylcarbonate, alkylsufonate, alkylsulfamate, and semipermeable polymer forming groups.

The semipermeable polymeric compositions typically include a member selected from the group consisting of cellulose acylate, cellulose diacylate, cellulose triacylate, cellulose acetate, cellulose diacetate, cellulose triacetate, and mono-, di- and tri-cellulose alkanylates, mono-, di- and tri-alkenylates, and mono-, di- and tri-aroylates. Exemplary polymers including cellulose acetate having a D.S. of 1.8 to 2.3 and an acetyl content of 32 to 39.9%; cellulose diacetate having a D.S. of 1 to 2 and an acetyl content of 21 to 35%; cellulose triacetate having a D.S. of 2 to 3 and an acetyl content of 34 to 44.8%. More specific cellulosic polymers include cellulose propionate having a D.S. of 1.8 and a propionyl content of 38.5%; cellulose acetate propionate having an acetyl content of 1.5 to 7% and an acetyl content of 39 to 42%; cellulose acetate propionate having an acetyl content of 2.5 to 2%, an average propionyl content of 39.2 to 45% and a hydroxyl content of 2.8 to 5.4%; cellulose acetate butyrate having a D.S. of 1.8, an acetyl content of 13 to 15%, and a butyryl content of 34 to 39%; cellulose acetate butyrate having an acetyl content of 2 to 29.5%, a butyryl content of 17 to 53%, and a hydroxyl content of 0.5 to 4.7%; cellulose triacylates having a D.S. of 2.9 to 3%, such as cellulose trivalerate, cellulose trilaurate, cellulose tripalmitate, cellulose trioctanoate, and cellulose tripropionate; cellulose diesters having a D.S. of 2.2 to 2.6%, such as cellulose disuccinate, cellulose dipalmitate, cellulose dioctanoate, and cellulose dicarpylate; mixed cellulose esters such as cellulose acetate valerate, cellulose acetate succinate, cellulose propionate succinate, cellulose acetate octanoate, cellulose valerate palmitate, and cellulose acetate heptonate. Semipermeable polymers are known in U.S. Pat. No. 4,077,407, and they can be made by procedures described in *Encyclopedia of Polymer Science and Technology*, Vol. 3, pages 325 to 354, 1964, published by Interscience Publishers, Inc., New York.

Additional semipermeable polymers for manufacturing wall 12 include acetaldehyde dimethyl cellulose acetate; cellulose acetate ethylcarbamate; cellulose acetate methylcarbamate; cellulose dimethylaminoacetate; semipermeable polyamides; semipermeable polyurethanes; semipermeable polyimides; semipermeable sulfonated polystyrenes; cross-linked, selectively semipermeable polymers formed by the coprecipitation of a polyanion and a polycation as disclosed in U.S. Pat. Nos. 3,173,876; 3,276,586; 3,541,005; 3,541,006; and 3,546,142; semipermeable polymer as disclosed by Loeb and Sourirajan in U.S. Pat. No. 3,133,132; semipermeable polystyrene derivatives; semipermeable poly(sodium styrenesulfonate); semipermeable poly(vinylbenzyltrimethyl)ammonium chloride; semipermeable polymers exhibiting a fluid permeability of 10 to 10⁻⁶ (cc.mil/cm.hr.atm) expressed as per atmosphere of hydrostatic or osmotic pressure difference across a semipermeable wall. The polymers are known the art in U.S. Pat. Nos. 3,845,770; 3,916,988; and 4,160,020, and in *Handbook of Common Polymers*, by Scott, JR and Roff, WJ, 1971, published by CRC Press, Cleveland, Ohio.

In a presently preferred optional manufacture, the external surface of wall 12 is coated with an enteric composition. The enteric composition is a means for delaying the passage of fluid through wall 12 especially in an acidic environment. The enteric composition does not dissolve, disintegrate, or change its structural nature in the stomach, and during the period of time dosage form 10 passes through the stomach. Representative of compositions that keep their integrity in the acidic environment of the stomach comprise: (a) a member selected from the group of phthalates consisting of cellulose acetyl phthalate, cellulose diacetyl phthalate, cellulose triacetyl phthalate, cellulose acetate phthalate, hydroxypropyl methylcellulose phthalate, sodium cellulose ether phthalate, cellulose ester phthalate, methylcellulose phthalate, cellulose ester-ether phthalate, hydroxypropyl cellulose phthalate, alkali salts of cellulose acetate phthalate, alkaline earth salts of cellulose acetate phthalate, calcium salt of cellulose acetate phthalate, ammonium salt of hydroxypropyl methylcellulose phthalate, cellulose acetate hexahydrophthalate, hydroxypropyl methylcellulose hexahydrophthalate, polyvinylacetate phthalate and the like; (b) a member selected from the group consisting of keratin, keratin sandaractolu, salol, salol betanaphthyl benzoate and acetotannin, salol with balsam of Peru, salol with tolu, salol with gum mastic, salol and stearic acid, and salol and shellac; (c) a member selected from the group consisting of formalized gelatin, and formalized cross-linked gelatin and exchange resins; (d) a member selected from the group consisting of myristic acid-hydrogenated castor oil-cholesterol, stearic acid-mutton tallow, stearic acid-balsam of tolu, and stearic acid-castor oil; (e) a member selected from the group consisting of shellac, ammoniated shellac, ammoniated shellac-salol, shellac-wool fat, shellac-acetyl alcohol, shellac-stearic acid-balsam of tolu, and shellac n-butyl stearate; (f) a member selected from the group consisting of abietic acid, methyl abietate, benzoin, balsam of tolu, sandarac, mastic with tolu, and mastic with acetyl alcohol; (g)a member selected from the group consisting of cellulose acetate phthalate with shellac, start acetate phthalate, polyvinyl acid phthalate, 2-ethoxy-5-(2-hydroxyethyxy)-methylcellulose phthalic acid, acid phthalates of carbohydrates, zein, alkylresin unsaturated fatty acids-shellac, colophony, mixtures of zein and carboxymethylcellulose phthalate; and (h) anionic polymers synthesized from methacrylic acid and methacrylic acid methyl ester, copolymeric acrylic resins of methacrylic acid and methacrylic acid methyl ester, copolymers of methacrylic acid and methacrylic acid methyl ester with diallyl phthalates, copolymers of methacrylic acid and methacrylic acid methyl ester with dibutyl phthalate. The acid resistance materials known in *Remington's Pharmaceutical Science,* (1965), 13th ed., pages 604-605, published by Mack Publishing Co., Easton, PA; *Eudragit*® *Coatings Rohm Pharma*, (1985); and U.S. Pat. No. 4,627,851.

Wall 12 surrounds an inner wall 19 that defines a capsule with an interior lumen 20 or ingredient receiving space 20. Wall 19 or capsule 19 generally is tubular shaped and in a presently preferred manufacture comprises a hemispherical or dome-shaped lead end 14. Capsule wall 19 of dosage form 10, in one manufacture, is made by dipping a mandrel, such as stainless-steel mandrel, into a bath containing solution of a capsule-wall forming material to coat the mandrel with the material. Then, the mandrel is withdrawn, cooled, and dried in a current of air. The capsule is stipped from the mandrel and trimmed to yield a capsule with an internal lumen. The capsule can be manufactured also by injection molding using a conventional molding machine. Representation of capsule forming compositions comprise the commercially available materials including gelatin, gelatin having a viscosity of 15 to 30 millipoises and a bloom strength up to 150 grams; gelatin having a bloom value of 160 to 250; a composition comprising gelatin, glycerine water and titanium dioxide; a composition comprising gelatin, erythrosin, iron oxide and titanium dioxide; a composition comprising gelatin, glycerine, sorbitol, potassium sorbate and titanium dioxide; a composition comprising gelatin, acacia, glycerin and water; water soluble polymers that permit the transport of water therethrough and can be made into capsules; and the like. The capsule can be formed also of a thiolated gelatin. The thiolated gelatin can be synthesized by reacting a thiolactone, such as a N-acetylhomocysteine thiolactone, with gelatin, whereby free sulfhydryl groups are attached through peptide bonds to the gelatin. The internal capsule can be manufactured as a simple piece or single unit capsule, or the capsule can be manufactured as a two-piece capsule. The synthesis is accomplished by following the synthesis of Benesch and Benesch, as reported in *Proc. Natt. Acad. Sci*., U.S., Vol 55(9), pages 848 to 1853, 1958. Procedures for manufacturing one-piece and two-piece capsules are known in U.S. Pat. No. 4,663,148; 4,663,149; 4,675,174; and 4,678,467.

Drawing Figure 4 thus presents dosage form 10 in opened view for illustrating the structure and the internal contents of dosage form 10. In drawing Figure 4, dosage form 10 comprises a body 11, a wall 12, an exit passageway 13, a lead end 14 and a rear end 15. Wall 12 is an exterior or outside wall that surrounds ad contacts an internal capsule wall 19. Inside capsule wall 19, is illustrated as a single section capsule in drawing Figure 4. Capsule wall 19 comprises an internal lumen 20, which lumen 20 can be filled prior to the application of wall 12, or lumen 20 can be filled during the manufacture of capsule 19, or lumen 20 can . be filled by injection after application of wall 12. Lumen 20 contains a non-aqueous lipophilic carrier represented by wavy lines 21, which carrier comprises a drug represented by dots 22.

The term drug 22 as used herein, denotes a medicament, a pharmacologically active composition of matter endowed with therapeutic properties, that can be delivered from dosage form 10 to produce a therapeutic result. In the specification and the accompanying claims, the term "drug" includes any physiologically or pharmacologically active substance that produces a local or a systemic effect in animals, including warm-blooded animals, humans, avian, household, sport and farm animals, laboratory and zoo animals. The term "physiologically", as used herein, denotes the administration of a drug to produce normal levels and functions. The term "pharmacologically", as used herein, denotes a beneficial response relative to the amount of drug administered to a drug receiving host, see Stedman's Medical Dictionary; 1966, published by Williams and Wilkins, Baltimore, MD. The phrase "drug formulation" as used herein means drug 22 is in lumen 20 mixed with a pharmaceutically acceptable carrier 21 and other drug-carrier formulation ingredients. The active drug 22 that can be delivered by dosage form 10 include inorganic and organic therapeutic compounds without limitation, including drugs that act on the peripheral nerves, adrenergic receptors, cholinergic receptors, nervous system, skeletal muscles, cardiovascular system, smooth muscles, blood circulatory system, synoptic sites, neuroeffector junctional sites, endocrine system, hormone systems, immunological system, organ systems, reproductive system, skeletal system, autocoid systems, alimentary and excretory systems, inhibitory of autocoids and histamine systems, and physiological systems. The active drug that can be delivered for acting on these animal systems incudes depressants, beta-blockers, hypnotics, calcium channel blockers, angiotensin converting enzyme inhibitors, immunosuppressants, sedatives, psychic energizers, tranquilizers, anti-convulsants, muscle relaxants, antiparkinson agents, anti-viral, analgesics, anti-inflammatories, local anesthetics, muscle contractants, anti-microbials, anti-malarials, hormonal agents, contraceptives, anti-hypertensive sympathomimetics, diuretics, antiparasitics, neo-plastics, hypoglycemics, ophthalmics, electrolytes, and diagnostic agents.

Drug 22, in one preferred embodiment, comprises a peptide or polypeptide. The term "peptide" as sued for the purpose of this invention, denotes a drug or composition of matter comprising an amino acid and more specifically, a drug or composition comprising two or more amino acids in which the alpha carboxyl group of one amino acid is united with the alpha amino acid group of another amino acid, to form a peptide bond. The term "polypeptide", for the purpose of the present invention denotes a peptide comprising a number of amino acids, that is, a molecular chain of therapeutically acceptable amino acids. The invention embraces dipeptides, tripeptides, and polypeptides. The term "polypeptides" incudes proteins, enzymes, nucleoproteins, glycoproteins, lipoproteins, hormonally active polypeptides, and synthetic analogs, including agonists and antagonists of these molecules. The proteins which are operable for the purpose of this invention comprise a member selected from the group consisting of immune modulators, lymphokines, monokines, cytokines, enzymes, antibodies, growth promotants, growth inhibitory factors, blood proteins, hormones, vaccines, viral antigen, bacterial antigen, parasitic antigen, rickettsial antigen, antibodies, precursor proteins, muteins, and the like.

Representative of peptides include corticotropin, calcitonin, growth factor, somatostatin, analogs, octreotide, secretin, vasoactive intestinal peptide, pancreatic enzyme, somatotropin, superoxide dismutase, tumor necrosis factor, thyrotropin releasing hormone, insulin, thyroid stimulating hormone, luteinizing hormone, colony stimulating factor, chorionic gonadotropin, calcitonin, gene related peptide, atrial natriuretic peptide, oxytocin, vasopressin, vasopressin analogs, erythroprotein, parathyroid hormone, parathyroid hormone analogs, enzyme inhibitors, trypsin inhibitors, chymostrysin inhibitors, aprotinin, bile sales, including deoxycholate, cholate, ursodeoxycholate, glycolate, taurocholate, taurodeoxycholate, luteinizing hormone releasing factor, include the following biologically active macromolecules, and muteins and other analogs thereof: interferons (α-, β-, γ-, and the like), interleukins (IL-1, IL-1α, IL-1β, II-2, IL-3, II-4, IL-5, and the like), macrophage activating factors, macrophage peptides, B cell factors (B cell growth factor and the like), T cell factors, protein A, suppressive factor of allergy, suppressor factors, cytoxic glycoprotein, immunocytotoxic agents, immunotoxins, immunotherapeutic polypeptides, lymphotoxins, tumor necrosis factors (*α-*, *β-*, and the like), cachectin, oncostatins, tumor inhibitory factors, transforming growth factors such as TGF-α and TGF-β), albumin, alpha-1-antitrypsin, apolipoprotein-ε, erythroid potentiating factors, erythropoietin, factor VII, factor VIII(c), factor IX, fibrinolytic agent, hemopoietin-1, kidney plasminogen activator, tissue plasminogen activator, urokinase, pro-urokinase, streptokinase, lipocortin, lipomodulin, macrocortin, lung surfactant protein, protein C, protein 5, C-reactive protein, renin inhibitors, collagenase inhibitors, platelet derived growth factor, osteogenic growth factors, atrial natruretic factor, auriculin, atriopeptin, bone morphogenic protein, calcitonin precursor, calcitonin gene-related peptide, cartilage inducing factor, connective tissue activator protein, fertility hormones, follicle stimulating hormone, growth hormone releasing factor, osteogenic protein, proinsulin, nerve growth factor, parathyroid hormone, parathyroid hormone inhibitors, relaxin, somatomedin C, inhibin, adrenocorticotrophic hormone, glucagon, gastric inhibitory peptide, motilin, cholecystokinin, pancreatic polypeptide, gastrin releasing peptide, corticotropin releasing factor, thyroid stimulating hormone, vaccine antigens, including antigens of HTLV-I, II, AIDS viruses such as HTLV-III/LAV/HIV and HIV-2, cytomegalovirus, hepatitis A < B and non-A/non-B, herpes simplex virus-I, herpes simplex virus II, malaria, pseudorabies, retroviruses, feline leukemia virus, bovine leukemia virus, transmissible gastroenteritis virus, infectious bovine rhinotracheitis, parainfluenza, rotaviruses, respiratory syncytial virus, varicella zoster virus. Epstein-Barr virus, pertussis, and anti-infective antibodies including monoclonal and polyclonal antibodies to gram negative bacteria, pseudomonas, endotoxin, and tetanus toxin.

Representative of other drugs 22 that can be dispensed by the dosage form of this invention includes cyclosporine, cyclosporin A, dihydrocyclosporin C, cyclosporin D, and dihydrocyclosporin D, as disclosed in U.S. Pat. Nos. 4,127,118 and 4,388, 307; captopril and its acceptable derivatives, zofenopril, fosinopril, enalapril; carboxyalkyl dipeptide angiotensin converting inhibitors, ether and thioether mercaptoacyl proline angiotensin converting inhibitors, enalaprilat, lisinopril, and the like. Examples of more specific angiotensin converting enzyme inhibitors comprise proline derivatives, such as any of those disclosed in U.S. Pat. No. 4,046,889 or 4,105,776 to Ondetti et al comprising captopril, that is, 1-(2S)-3-mercapto-2-methylpropionyl]-L-proline, being preferred, carboxyalkyl dipeptide derivatives, such as any of those disclosed in U.S. Pat. No. 4,374,829 including N-(1-ethoxycarbonyl-3-phenylpropyl)-L-alanyl-L-proline, that is, enalapril. Other examples of angiotensin converting enzyme, ACE, inhibitors suitable for use herein, include any of the phosphonate substituted amino or imino acids or salts disclosed in U.S. Pat. No. 4,452,790 such as (S)-1-[6-amino-2-[hydroxy-4-phenylbutyl)phosphinyl]oxy]-1-oxoheyxl]-L-proline, phosphinylalkanoyl prolines disclosed in U.S. Pat. No. 4,168,267 such as fosinopril, mercaptoacyl derivatives of substituted prolines, disclosed in U.S. Pat. No. 4,316,906 such as zofenopril or any of the phophinylalkanoyl substituted prolines disclosed in U.S. Pat. No. 4,337,201 and the phosphonamides disclosed in U.S. Pat. No. 4,432,971. Other examples of ACE inhibitors include Beecham's BRL 36,378 as disclosed in European Pat. Nos. 80822 and 60668; Chugai's MC-838 disclosed in CHEM. ABST. 102:72588v and Jap. J. Pharmacol. 40:373 (1986); Ciba-Geigy's CGS 14824 (3-[1-ethoxycarbonyl-3-[phenyl-(1 S)-propyl)amino)-2,3,4,5-tetrahydro-2-oxo-1 (3S)-benzazepine-1 acetic acid HCI) disclosed in U.K. Pat. No. 2103614 and CGS 16,617 (3(S)-5-amino-1-carboxypentyl]amino]-2,3,4,5-tetrahydro-2-oxo-1H-1-benzazepine-1-ethanoic acid) disclosed in U.S. Pat. No. 4,473,575; cetaphil (alacepril, Dainippon) disclosed in Eur. Therap. Res. 39:671 (1986); 40:543 (1986); ramipril (Hoechst) disclosed in Arzneimittelforschung 35:1254 (1985); cilazapril (Hoffman-LaRoche) disclosed in J. Cardiovasc. Pharmacol. 9:39 (1987); Rₒ 31-2201 (Hoffman-LaRoche) disclosed in FEBS Lett. 165:201 (1984); lisinopril (Merck) disclosed in Curr. Therap. Res. 37:342 (1985) and Eur. Pat. appln. No. 21-401, indolapril (delapril) disclosed in Clin. Exp. Pharmacol. Physiol. 5:643,655 (1983); non-sulfhydryl angiotensin converting enzyme inhibitors, sulfhydryl angiotensin converting enzyme inhibitors, spirapril (Schering) disclosed in Acta Pharmacol. Toxicol. 59(Supp. 5):173 (1986); delapril, perindopril, (Servier) disclosed in Eur. J. Clin. Pharmacol. 31:519 (1987); imidapril, cilazapril, cilazaprilat, sperapril, quinapril (Warner-Lambert) disclosed in U.S. Pat. No. 4,344,949 and Cl 925 (Warner-Lamber)(3S-[2[R(*)R(*)]]3R(*)]-2]2-[[1-eyhoxycarbonyl)-3-phenylpropylamino[-1-oxopropyl]-1,2,3,4,-tetrahydro-6,7-dimethoxy-3-isoquinolinecarboxylic acid HCI) disclosed in Pharmacologist 26:243, 266 (1984), WY-44221 (Wyeth) disclosed in J. Med. Chem., 26:394 (1983), and the angiotensin converting enzyme inhibitors disclosed in U.S. Pat. No. 4,931,430.

Examples of additional drugs 22 that can be delivered by dosage form 10 comprise progestins, estrogenic, progestational, corticosteroids, hydrocortisone, hydrocorticosterone acetate, cortisone acetate, triamcinolone, methylesterone, 17 beta-estradiol, ethinyl estradiol, ethinyl estradiol 3-methyl ester, prednisolone, 17-beta-hydroxyprogesterone acetate, 19-nor-progesterone, norgestrel, norethindrone, norethisterone, morethiederone, progesterone, norgesterone, norethynodrel, indomethacin, naproxen, fenoprofen, sulindac, indoprofen, nitroglycerin, isosorbide dinitrate, propranolol, timolol, atenolol, alprenolol, cimetidine, clonidine, imipramine, levodopa, chlorpromazine, methyldopa, dihydroxyphenylalanine, pivaloyloxyethyl ester of alpha-methyldopa hydrochloride, theophylline, calcium gluconate, ketoprofen, ibuprofen, cephalexin, erythromycin, haloperidol, zomepirac, ferrous lactate, vincamine, diazepam, phenoxybenzamine, diltiazem. milrinone, captopril, mandol, guanabenz, hydrochlorothiazide, ranitidine, cimetidine, flurbiprofen, fenbufen, fluprofen, tolmetin, alclofenac, mefenamic, flufenamic, difuninal, nimodipine, nitredipine, nifedipine, nisoldipine, isradipine, nicardipine, felodipine, lidoflazine, tiapamil, gallopamil, amlodipine, mioflazine, ramipril, endlapriat, famotidine, nizatidine, sucralfate, etintidine, tertatolol, minoxidil, chlordiazepoxide, chlordiazepoxide hydrochloride, diazepam, amitriptyline hydrochloride, imipramine hydrochtoride, imipramine pamoate, and the like. The drugs are known to the art in Pharmaceutical Sciences, 14th ed., edited by Remington, (1979J published by Mack Publishing Co., Easton, PA; The Drug, The Nurse, The Patient, Including Current Drug Handbook, by Falconer, et al (1974-1976) published by Saunder Company, Philadelphia, PA; Medicinal Chemistry, 3rd ed., Vol. 1 and 2, by Burger, published by Wiley-Interscience, New York; and in Physician's Desk Reference, 38th ed., (1984) published by Medical Economics Co., Oradell, NJ. The amount of beneficial drug in a dosage form generally is about from 0.05 ng to 5 g or more, with a preferred dose of 0.05 ng to 1.5 g, with individual devices containing, for example, 25 ng, 1 mg, 5 mg, 10 mg, 25 mg, 125 mg, 250 mg, 500 mg, 750 mg, 1.0 g. 1.2 g, 1.5 g, and the like. The dosage form can be administered once, twice or thrice daily.

The pharmaceutically acceptable carrier 21 may be a non-aqueous lipophilic carrier which comprises a glyceride 23 represented by a horizont line 23, which glyceride are esters are glycerol and a fatty acid in which one or more of the hydroxyl groups of the glycerol have been replaced by an acidic moiety. The fatty acid can be saturated or unsaturated fatty acid. The saturated fatty acid comprises from 4 to 26 carbon atoms and the unsaturated fatty acid comprises 10 to 24 carbon atoms. Fatty acids are disclosed in Organic Chemistry, by Fieser and Fieser, pages 382 to 383, (1944), published by D.C. Heath and Company. The glycerides comprises a member selected from the group consisting of monoglycerides, diglycerides and triglycerides. Specific glycerides 23 operable for the present invention comprise a member selected from the group consisting of glyceryl monobutyrate, glyceryl monoisovalerate, glyceryl monothiglycolate, glyceryl monocorate, glyceryl monooleate, glyceryl monorunoleate, glyceryl monoisolalerate, glyceryl monocaporate, glyceryl momoyristate, glyceryl monopalmitate, glyceryl monostearate, glyceryl monoarachidate, glyceryl monobehenate, glyceryl monolignocerate, glyceryl monocerotate, glyceryl dialurate, glyceryl dioleate, glyceryl distearate, glyceryl dihydroxystearate, glyceryl dilanolate, glyceryl dilaurate, glyceryl dilaurate, glyceryl dilionleate, glyceryl oleate, glyceryl stearate, glyceryl esters of mixed acids, glyceryl esters of hydrogenated cottonseed oil, glyceryl triester of coconut oil acids, glyceryl triester of hydrogenated soybean oil acids, and glyceryl triester of lard acids. Additional carrier 21 glycerides 23 include succinylated monoglyceride, acetylated monoglyceride, glyceride of palm oil, glyceride of vegetable oils, and tartaric acid ester of monoglyceride. The concentration of glyceride in carrier 21 is from 1 wt % to 60 wt %..

Carrier 21 comprises additionally a hydrogenated oil represented by vertical line 24. Representative of hydrogenated oil comprises a member selected from the group consisting of hydrogenated vegetable, hydrogenated plant, hydrogenated fruit, hydrogenated wood, hydrogenated nut, and hydrogenated fish and hydrogenated sperm oils. Further representatives of hydrogenated oils include hydrogenated wormwood oil, hydrogenated almond oil, hydrogenated rosemary oil, hydrogenated bayberry oil, hydrogenated castor oil, hydrogenated peanut oil, and hydrogenated sperm oil. The amount of hydrogenated oil in carrier 21 is from 1 wt % to 60 wt %.

Carrier 21 comprises also a glycol represented by slanted lines 25. The glycols comprise liquid glycols, ethylene glycol, propylene glycol, butylene glycol, isopropylene glycol, disopropylene glycol, trimethylene glycol, polyethylene glycol, polyalkylene glycol, ethylene glycol monomethyl ether, propylene glycol esters of fatty acids such as hexanoic, octanoic acid decanoic, dodecanoic, caproic, caprylic, capric and lauric, dialkylated glycol, triethylated glycol and hexylene glycol. The amount of glycol present in carrier 21 is 1 wt % to 60 wt %, with the amount of all ingredients in carrier 21 is equal to 100 wt %.

Drawing Figure 4 also illustrates a driving means 26 that possesses osmotic properties. Driving means 26 is an osmotic composition that is activated in a fluid environment. In one embodiment, osmotic composition 26 comprises an osmotic solute that exhibits and osmotic pressure gradient across wall 12 against an external fluid present in the environment of use. In another embodiment, osmotic composition 26 comprises a fluid activated hydrogel that imbibes and/or absorbs fluid into osmotic composition 26 through outer semipermeable wall 12 thereby increasing in volume, expanding and pushing the drug carrier formulation through exit 13 from dosage form 10. Semipermeable wall 12 is non-toxic, it maintains its physical and chemical integrity during operation of dosage form 10; and wall 12 is free of any interaction with osmotic composition 26. The osmotic composition 26 comprises in one manufacture 2 wt % to 50 wt % of an osmotically effective solute, in another manufacture 10 wt % to 75 wt % of an osmotic hydrogel, an in another embodiment a blend of an osmotic solute and an osmotic hydrogel.

Composition 26 comprises an osmotically effective compound that imbibes fluid into the dosage form, thereby making available hydro-energy for pushing against a pharmaceutically acceptable carrier 21 for delivery carrier 21 from dosage form 10. The osmotically effective compounds are known also as osmotically effective solutes, and also as osmagents. Osmotically effective solutes, used for forming composition 26, comprise magnesium sulfate, magnesium chloride, potassium sulfate, sodium sulfate, lithium sulfate, sodium chloride, potassium acid phosphate, mannitol, urea, inositol, magnesium succinate, tartaric acid, carbohydrates such as raffinose, sucrose, glucose, lactose, sorbitol, potassium chloride and mixtures therefor.

Representative of hydro-activated hydrogels, also known as osmopolymers for formulating composition 26 are osmopolymers that exhibit fluid imbibition properties. The osmopolymers are swellable, hydrophilic polymers, which osmopolymers interact with water and biological aqueous fluids and swell or expand to an equilibrium state. The osmopolymers exhibit the ability to swell in water and biological fluids and retain a significant portion of the imbibed fluid with the polymer structure. The osmopolymers swell or expand to a very high degree, usually exhibiting a 2 to 50 fold volume increase. The osmopolymers can be noncross-linked or cross-linked. The swellable, hydrophilic polymers are in one preferred embodiment lightly cross-linked, such cross-links being formed by covalent or ionic bonds or residue crystalline regions after swelling. The osmopolymers are hydrophilic polymers. Hydrophilic osmotic polymers suitable for the present purpose include poly-(hydroxyalkyl methacrylate) having a molecular weight of from 20,000 to 5,000,000; poly(vinylpyrrolidone) having a molecular weight of from 10,000 to 360,000; anionic and cationic hydrogels; polyelectrolyte complexes; poly(vinyl alcohol) having a low acetate residual, cross-linked with glyoxal, formaldehyde, or glutaraldehyde and having a degree of polymerization of from 200 to 300,000; a mixture of methyl cellulose, cross-linked agar and carboxymethyl cellulose; a mixture of hydroxypropyl methylcellulose and sodium carboxymethylcellulose; a mixture of hydroxypropyl methylcellulose and sodium carboxymethylcellulose; a mixture of hydroxypropyl ethylcellulose and sodium carboxymethyl cellulose; sodium carboxymethylcellulose; potassium carboxymethylcellulose; a water insoluble, water swellable copolymer formed from a dispersion of finely divided copolymer of maleic anhydride with styrene, ethylene, propylene, butylene or isobutylene cross-linked with from 0.001 to about 0.5 moles of saturated cross-linking agent per mole of maleic anhydride per copolymer; water swellable polymers of N-vinyl lactams; polyoxyethylene-polyoxypropylene gel; polyoxybutylene-polyethylene block copolymer gel; carbo gum; polyacrylic gel; polyester gel; polyuria gel; polyether gel; polyamide gel; polycellulosic gel; polygum gel; initially dry hydrogels that imbibe and absorb water which penetrates the glass hydrogel and lowers is glass temperature.

Representatives of other osmopolymers for providing composition 26 are for example, osmopolymers known as hydrogels such as Carbopol® acidic carboxypolymer, a polymer of acrylic and cross-linked with a polyallyl sucrose, also known as carboxypolymethylene and carboxyvinyl polymer having a molecular weight of 250,000 to 4,000,000; Cyanamer® polyacrylamides; cross-linked water swellable indene-maleic anhydride polymers; Good-rite® polyacrylic acid having a molecular weight of 100,000; Polyox® polyethylene oxide polymer having a molecular weight of 100,000 to 7,500,000 or higher; starch graft copolymers; Aqua-Keps® acrylate polymer polysaccharides composed of condensed glucose units such as dieters cross-linked polygluran; and the like. Representative polymers that provide hydrogels are known to the prior art in U.S. Pat. No. 3,865,108 issued to Hartop; U.S. Pat. No. 4,002,173 issued to Manning; U.S. Pat. No. 4,207,893 issued to Michaels; and in Handbook of Common Polymers, Scott and Roff, published by the Chemical Rubber Co., Cleveland, Ohio. Osmotically effective salutes are known to the prior art in U.S. Pat. No. 4,783,337.

Drawing Figure 5 depicts another improvement provided by the invention. The improvement comprises an osmotic barrier layer 27 positioned between pharmaceutically acceptable carrier 21 and osmotic composition 26, which barrier layer 27 substantially prevents the mixing, or the penetration of carrier 21 into osmotic composition 26. In this manufacture, osmotic barrier layer 27 also assures the driving power of osmotic composition 26 will be applied against carrier 21 to ensure that a maximum amount of drug is delivered from dosage form 10. Barrier layer 27 is made from a substantially fluid impermeable composition comprising a polymeric composition, high density polyethylene, wax, rubber, styrene butadiene, rubber, polysilicone, nylon, teflon, polystyrene, polytetrafluoroethylene, halogenated polymers, and a blend of a microcrystalline high acetyl cellulose and a high molecular weight fluid impermeable polymer.

The expression "exit means 13", as used herein, comprises means and methods suitable for the metered release of beneficial drug 22 from lumen 20 of dosage form 10. The exit means 13 includes at least one passageway or orifice through wall 12 for communicating with lumen 20. The expression "at least one passageway" includes aperture, orifice, bore, pore, porous element through which the drug can migrate, hollow fiber, capillary tube, porous overlay, or porous insert. The expression also includes a material that erodes or a material that is leached from wall 12 in the fluid environment of use to produce at least one passageway 13 in dosage form 10. Representative materials suitable for forming at least one passageway, or a multiplicity of passageways, include an erodible poly(glycolic) acid or erodible poty(lactic) acid member in the wall; a gelatinous filament; poly(vinyl alcohol); leachable materials such as fluid removable pore forming polysaccharides, saccharides, salts, oxides, and the like. A passageway or a plurality of passageways can be formed by leaching a material such as sorbitol, lactose, or the like, from the wall. The passageway can have any shape such as round, triangular, square, elliptical, and the like, for assisting in the metered release of drug 20 from dosage form 10. Dosage form 10 can be constructed with one or more passageways in spaced apart relations, or more than one passageway on a single surface *of* dosage form 10. Passageways and equipment for forming passageways are disclosed in U.S. Pat. Nos. 3,845,770; 3,916,899; 4,063,064; and 4,088,864. Passageways for releasing a drug formed by leaching are disclosed in U.S. Pat. Nos. 4,200,098 and 4,285,987.

The dosage form is manufactured by using the procedures that follows. All osmotic layer 26 components are passed through a sizing screen or sieve, then, the screened components are placed in a mixing container and blended into a homogenous blend. Next, a solvent, such as ethanol anhydrous is slowly added to the dry blend while mixing continued, until a wet mass suitable for screening is formed. The wet mass is passed through a screen to provide wet screened particles. The wet particles are dried until the moisture is evaporated and the dried particles are again passed through a sizing screen. Next, a lubricant such as magnesium stearate is blended with the dry osmotic granulation.

The barrier layer 27 is prepared by blending a microfine wax and a polyethylene oxide of 7,000,000 molecular weight and then passed through a sizing screen. The screened components are placed in a blender and blended to a homogenous mass. Next, a solvent, ethanol is added slowly to the dry blend while mixing it continuously until a wet mass is formed and then screened to provide wet screened particles. Next, the wet particles are dried and passed through a screen to provide dry particles.

The core comprising the drug composition and the barrier layer are compressed into bilayer osmotic tablet cores. A manual hydraulic press or a rotary press can be used for this manufacturing step with standard tableting procedures.

Next, gelatin capsules are separated into their short and long segments. The bilayer osmotic cores are inserted into the open end of the long gelatin capsule segment, with the barrier layer first. An insertion guide is used to reproducibly insert the cores a fixed distance into the capsule segments.

Next, a coating is prepared,by placing two solvents acetone and purified water into separate containers. Then, sorbitol and polyethylene glycol are added to the purified water to produce homogenous solutions. Next, cellulose acetate comprising an acetyl content of 39.8% is added to the acetone solvent. Then, both solutions are mixed until all solute components are in solution. Next, the aqueous solution is slowly added to the acetone solution with mixing, and mixing continued until a clear homogenous solution is produced. The solution is applied uniformly over the surface of coating assemblies using air suspension film-coating equipment. The solvent is evaporated during the process and the cellulose acetate polymers remained uniformly deposited on the systems.

Next, the systems are evenly distributed on drying trays and placed in an oven. After the systems are dried, the systems are placed into holding racks. The weights of unfilled systems are recorded before filling to allow subsequent tracking of drug solution fill weights. Then, the systems are filled with a drug solution, for example, a cyclosporin drug formulation, using either a semi-automatic filling pump, or a hand held syringe. During filling, the fill needle punctured the rounded end of the system forming a drug release orifice. The gelatin capsules are next separated into short and long segments. Then, the monoglyceride myverol, is melted and filled systems are dipped orifice end down into the melted monoglycerides to a depth of 3 to 4 mm. While holding the systems inverted, the orifice end of the systems are capped with the short segment of a size 0 gelatin capsule.

Following the above procedure a dispenser 10 is manufactured comprising a drug formulation, which formulation weights 560 mg and comprises 10 wt % cyclosporin, 36 wt % of a hydrogenated castor oil, 36 wt % of an aminodiglyceride, and 18 with propylene glycol. An amino triglyceride can be used at this step too. The barrier layer in the dispense weight 50 mg comprising 90 wt % microcrystalline wax and 10 polyethylene oxide possessing a 7,500,000 molecular weight; an osmotic push layer that weight 300 mg and comprises 59.75 wt % of polyethylene oxide possessing a 7,500,000 molecular weight, 29 wt % sodium chloride, 5 wt % carbopol-934-P, a carboxyvinyl polymer, 5 wt % hydroxypropyl-methylcellulose possessing a 9,200 molecular weight, 1 wt % ferric oxide and 0.25 wt % magnesium stearate. The dispenser is manufactured with a semipermeable wall 50 to 70 mg in weight and consisting of a wall composition comprising 65 wt % cellulose acetate having a 39.8 wt % acetyl content, 20 wt % sorbitol and 15 wt % polyethylene glycol, and a wall composition comprising 85 wt % cellulose acetate having an 39.8% acetyl content, 10 wt % sorbitol and 5 wt% polyethylene glycol. The gelatin capsule weight 80 mg and consists of 100 wt % gelatin. The dispenser comprises a 30 mg orifice cap seal, consisting of 100 wt % Myverol.
The accompanying drawing Figure 6 depicts the cumulative release of cyclosporin from two dispensers provided by the invention. In drawing Figure 6, the cumulative cyclosporin released in mg versus time is plotted with a semipermeable wall weighing 70 mg and seen as clear squares, and a semipermeable wall weighing 50 mg, is seen in shaded squares, as measured in artificial gastric juice.

### DOSAGE FORM OF THE INVENTION

One embodiment of the invention pertains to a dosage form for delivering a drug to the gastrointestinal tract of a human at a rate controlled by a delivery system over an extended period of time up to 36 hours, which allows the steps of: (A) admitting orally into the gastrointestinal tract a dosage form comprising: (1) a semipermeable wall comprising an inside surface that surrounds and forms an internal compartment, said semipermeable wall comprising a composition permeable to the passage of a biological fluid; (2) an inside wall comprising a protein on contact with the inside surface of the semipermeable wall; (3) a drug comprising a member selected from non-peptide and peptide drugs in the compartment for delivering to the gastrointestinal tract; (4) push means in the compartment for pushing the drug from the dosage form; (5) passage means in the dosage form of delivering the drug from the dosage for; (B) imbibing fluid through the semipermeable wall into the compartment at a rate determined by the permeability of the semipermeable wall and the osmotic pressure gradient across the wall, thereby causing the push means to expand and push the drug from the dosage form; and (C) delivering the drug from the compartment by the expandable push means continuously expanding thereby causing the drug to be dispensed through the passageway at a therapeutically effective dose at a rate controlled over an extended period of time to the gastrointestinal tract of the method.
The dosage form is also adapted to administer a drug buccally, sublingually, vaginally or rectally by using the dosage form of the invention.

## Claims

1. A dosage form (10) for the administration of a non-aqueous lipophilic liquid drug composition (22) to an environment of use, wherein the dosage form comprises:
(a) an outside wall (12) comprising a polymer semipermeable to the passage of a biological fluid and substantially impermeable to the passage of the drug;
(b) an inside wall (19) in contact with outside wall (12), said inside wall (19) comprising a protein polymer;
(c) a compartment (20) formed by the outside and the inside wall together;
(d) a hydrophilic push composition (26) in the compartment;
(e) an exit (13) in the outside and inside walls that connects the exterior with the inside of the dosage form;
(f) a non-aqueous lipophilic liquid drug (22) composition in the compartment;
(g) said liquid drug composition comprising a drug and a carrier (21) for the drug comprising a member selected from a hydrogenated glyceride, a monoglyceride, a diglyceride, and a triglyceride; a member selected from a glycol, a polyalkylene glycol and an ester of a glycol; and a hydrogenated oil;
**characterised in that** there is provided a substantially fluid impermeable barrier layer (27) positioned between the push composition (26) and the drug composition (22) and **in that** the drug is a non-aqueous liquid at both room temperature and at mammalian temperature to facilitate delivery of a therapeutically effective dose therefrom over time.

## Patentansprüche

1. Dosierungsform (10) zur Verabreichung einer nicht-wässrigen, lipophilen, flüssigen Arzneimittelzusammensetzung (22) an eine Einsatzumgebung, worin die Dosierungsform umfaßt:
(a) eine Außenwand (12), welche ein Polymer umfaßt, das gegenüber dem Durchfluß einer biologischen Flüssigkeit semipermeabel ist und gegenüber dem Durchfluß des Arzneimittels praktisch undurchlässig ist;
(b) eine Innenwand (19), die mit der Außenwand (12) in Kontakt steht, worin die Innenwand (19) ein Proteinpolymer umfaßt;
(c) eine Kammer (20), die durch die Außenwand und die Innenwand zusammen gebildet wird;
(d) eine hydrophile Ausstoßzusammensetzung (26) in der Kammer;
(e) ein Ausgang (13) in den Außen- und Innenwänden, die die Außenseite mit dem Inneren der Dosierungsform verbindet;
(f) eine nicht-wässrige, lipophile, flüssige Arzneimittelzusammensetzung (22) in der Kammer;
(g) wobei die flüssige Arzneimittelzusammensetzung ein Arzneimittel und einen Träger (21) für das Arzneimittel umfaßt, welcher ein Mitglied; ausgewählt aus einem hydrierten Glycerid, einem Monoglycerid, einem Diglycerid und einem Triglycerid; ein Mitglied, ausgewählt aus einem Glycol, einem Polyalkylenglycol und einem Ester eines Glycols; und ein hydriertes Öl umfaßt,
**dadurch gekennzeichnet, daß** eine praktisch flüssigkeitsundurchlässige Barriereschicht (27) zur Verfügung gestellt wird, die zwischen der Ausstoßzusammensetzung (26) und der Arzneimittelzusammensetzung (22) angeordnet ist, und dadurch, daß das Arzneimittel eine nicht-wässrige Flüssigkeit sowohl bei Raumtemperatur als auch bei der Temperatur eines Säugers ist, um die Abgabe einer therapeutisch effektiven Dosis daraus über einen bestimmten Zeitraum zu erleichtern.

## Revendications

1. Forme posologique (10) pour l'administration d'une composition de médicament liquide lipophile non aqueuse (22) à un environnement d'utilisation, la forme posologique comprenant :
(a) une paroi externe (12) comprenant un polymère semi-perméable au passage d'un fluide biologique et sensiblement imperméable au passage du médicament ;
(b) une paroi interne (19) en contact avec la paroi externe (12), ladite paroi interne (19) comprenant une protéine polymère ;
(c) un compartiment (20) formé par les parois externe et interne rassemblées ;
(d) une composition de poussée hydrophile (26) dans le compartiment ;
(e) une sortie (13) dans les parois externe et interne qui connecte l'extérieur avec l'intérieur de la forme posologique ;
(f) une composition de médicament liquide lipophile non aqueuse (22) dans le compartiment ;
(g) ladite composition de médicament liquide comprenant un médicament et un véhicule (21) pour le médicament comprenant un élément choisi parmi un glycéride hydrogéné, un monoglycéride, un diglycéride et un triglycéride ; un élément choisi parmi un glycol, un polyalkylène glycol et un ester d'un glycol ; et une huile hydrogénée ;
**caractérisée par le fait qu'**il est prévu une couche barrière (27) sensiblement imperméable aux fluides, positionnée entre la composition de poussée (26) et la composition de médicament (22), et **par le fait que** le médicament est un liquide non aqueux à la fois à la température ambiante et à la température du mammifère pour faciliter l'administration d'une dose thérapeutiquement efficace à partir de celui-ci au cours du temps.
